(19) **Europäisches Patentamt**
**European Patent Office**
**Office européen des brevets**

(11) **EP 0 942 284 B1**

(12) **EUROPÄISCHE PATENTSCHRIFT**

(45) Veröffentlichungstag und Bekanntmachung des Hinweises auf die Patenterteilung:
**30.01.2008 Patentblatt 2008/05**

(51) Int Cl.:
***G01N 33/86*** (2006.01)

(21) Anmeldenummer: **99102672.5**

(22) Anmeldetag: **12.02.1999**

(54) **Gebrauchsfertiges Prothrombinzeitreagenz auf der Basis von rekombinantem Gewebsfaktor**

Ready-to-use prothrombin time reagent based on recombinant tissue factor

Réactif prêt à l'emploi pour la détermination du temps de prothrombine à base d'un facteur tissulaire recombiné

(84) Benannte Vertragsstaaten:
**AT BE CH DE ES FR GB IT LI LU NL SE**

(30) Priorität: **13.03.1998 DE 19811016**

(43) Veröffentlichungstag der Anmeldung:
**15.09.1999 Patentblatt 1999/37**

(73) Patentinhaber: **Dade Behring Marburg GmbH**
**35001 Marburg (DE)**

(72) Erfinder: **Zander, Norbert Dr.**
**35037 Marburg (DE)**

(56) Entgegenhaltungen:
**EP-A- 0 585 987          WO-A-98/48283**
**US-A- 3 522 148          US-A- 5 314 695**

**Beschreibung**

Die vorliegende Erfindung betrifft ein gebrauchsfertiges langzeitstabiles Prothrombinzeitreagenz auf der Basis von rekombinantem Gewebsfaktor und dessen Verwendung in Gerinnungstests.

Die Prothrombinzeit (PT) ist der gängigste Screeningtest im Bereich der Gerinnungsdiagnostik. Hierbei wird Patientenplasma mit einem Reagenz gemischt, das mindestens Gewebsfaktor, Phospholipide und Calcium enthält. Der Gewebsfaktor kann aus Gewebe isoliert werden oder rekombinant hergestellt werden (Hoppenstaedt, D.A. et al. (1995) Lab.Med. 26(3), 198-203); dieses Protein aktiviert den extrinsischen Reaktionsweg der Gerinnung. Die gemessene Gerinnungszeit hängt von der Konzentration der Gerinnungsfaktoren II, V, VII und X ab. Die Auswertung der PT erfolgt in Sekunden Gerinnungszeit oder in Internationalen Normierten Ratios (INR). Die INR berechnet sich als $PR^{ISI}$, wobei die Prothrombinratio PR der Quotient aus der Gerinnungszeit der Probe und einer mittleren normalen Gerinnungszeit und der Internationale Sensitivitätsindex ISI eine Konstante ist, die vom Reagenz und dem verwendeten Meßgerät abhängt. Die Sensitivität eines Reagenzes ist umso größer, je numerisch kleiner der ISI ist. Ein ISI zwischen 0.9 und 1.2 ergibt eine optimale Sensitivität für Störungen des extrinsischen Systems. Noch geringere ISI-Werte führen zu extrem langen Gerinnungszeiten im pathologischen Bereich, die für viele Meßsysteme nicht mehr gut meßbar sind.

Die PT wird eingesetzt:

- als Screnningtest für das extrinsische Gerinnungssystem
- zur Kontrolle der oralen Antikoagulation
- zur Diagnostik von Lebererkrankungen.

Üblicherweise werden die meisten PT-Reagenzien, die erhältlich sind, in gefriergetrockneter Form angeboten und vor Gebrauch mit einem Rekonstitutionsmedium (in der Regel destilliertes Wasser oder eine Salzlösung) rekonstituiert. Der Grund hierfür ist die mangelnde Stabilität der Reagenzien im flüssigen Zustand.

Adam & Eberhard (US 3,522,148) beschreiben ein flüssiges Thromboplastinreagenz auf der Basis von Kaninchenhirnthromboplastin. Über die Sensitivität des Reagenzes werden keine Aussagen gemacht.

Butler et al. (US 5,385,853) beschreiben die Herstellung eines PT-Reagenzes aus Kaninchenhirnthromboplastin. Erfindungsgemäß ist die Zugabe von Polyethylenglykol (PEG) und die Verwendung von Calciumgluconat als Calciumquelle und Gentamycin als antimikrobielles Agenz. Der erzielte ISI-Wert ist allerdings mit im Mittel 2.0 sehr hoch; dies korrespondiert mit einer unzureichend niedrigen Sensitivität des Reagenzes.

Brown (US 5,314,695) beschreibt die Herstellung eines PT-Reagenzes auf der Basis von natürlichem oder rekombinantem Gewebsfaktor. Durch Zugabe bestimmter Phospholipidmischungen werden sensitive PT-Reagenzien hergestellt, die zur Lagerung jedoch bevorzugt lyophilisiert werden.

Zwei flüssige Thromboplastinreagenzien sind auf dem Markt erhältlich, eins von Pacific Hemostasis (USA), das andere von Diamed (Basel, Schweiz). Beide Reagenzien beruhen auf Kaninchenhirnthromboplastin, weisen eine geringe Sensitivität auf (ISI > 1.6) und haben eine Laufzeit von 1 Monat bzw. 1 Jahr.

Drei verschiedene rekombinate PT-Reagenzien sind auf dem Markt. Alle sind hoch sensitiv (ISI ca. 1.0) und werden in gefriergetrockneter Form angeboten.

Beschrieben wurden somit bereits folgende Verfahren :

- konventionelle gefriergetrocknete Thromboplastinreagenzien
- konventionelle gebrauchsfertige Kaninchenhirnthromboplastin-Reagenzien mit geringer Sensitivität
- rekombinante gefriergetrocknete Thromboplastinreagenzien mit hoher Sensitivität.

Mit den bisher bekannten Verfahren ist es bisher noch nicht gelungen, rekombinantes Thromboplastin so zu stabilisieren, daß es in einem Flüssigreagenz eingesetzt werden kann.

Die der vorliegenden Erfindung zugrunde liegende Aufgabe bestand daher darin, eine langzeitstabile Flüssigformulierung eines rekombinanten Thromboplastinreagenz bereit zu stellen. Ein solches Reagenz ist für das Gerinnungslabor außerordentlich vorteilhaft, da es die Vorteile der leichten Handhabbarkeit (Gebrauchsfertigkeit) mit den Vorteilen der hohen Sensitivität und guten Reproduzierbarkeit kombiniert.

Überraschenderweise wurde gefunden, daß eine Kombination eines Antioxidans in hoher Konzentration mit einem Serumalbumin in ebenfalls hoher Konzentration erfolgreich ist, während die einzelnen Stabilisatoren nicht hinreichend effektiv sind. Besonders vorteilhaft ist die Kombination von Ascorbinsäure mit Humanserumalbumin, jeweils in millimolarer Konzentration. Geringere Konzentration an einer der beiden Substanzen führte zu einer jeweils geringeren Stabilität.

Entscheidend für die Brauchbarkeit eines solchen Reagenzes ist die Stabilität bei ca. 4°C. Vorteilhaft ist eine Stabilität von mindestens 12 Monaten bei 4°C, besonders vorteilhaft eine Stabilität von mindestens 18 Monaten, ganz besonders vorteilhaft eine Stabilität von mindestens 24 Monaten.

Als Stabilität wird hier die zeitliche Konstanz des Meßergebnisses für die Bestimmung der PT eines definierten Plasmas, z.B. eines Normalplasmas definiert. Als konstant gilt das Meßergebnis, wenn es weniger als 10, bevorzugterweise weniger als 5 % abnimmt.

Um eine schnelle Information über die Stabilität bei 4°C zu gewinnen, erweist es sich in der Praxis als günstiger, die Reagenzien zunächst einer Streßbelastung bei 37°C zu unterwerfen.

Die Stabilität eines biologischen Materials läßt sich aus diesen Belastungsexperimenten abschätzen. Es gilt die Gleichung:

$$(1) \qquad A_t = A_o \, e^{-kt}$$

mit $A_t$ = Aktivität zum Zeitpunkt t, $A_o$ = Aktivität zu Beginn, t = Zeit und k = Degradationsgeschwindigkeitskonstante. Die Konstante k ist abhängig von der Temperatur. In Näherung gilt die Arrhenius - Gleichung:

$$(2) \qquad \log k = const\ 1 + [const\ 2\ /\ T]$$

mit T = absolute Temperatur.

In der Praxis wird die Stabilität etwa bei 4° C durch ein Belastungsexperiment, etwa bei 37° C, abgeschätzt. Als stabil gelte etwa eine Präparation, deren Aktivität um weniger als 5 % abgefallen ist. Für den entsprechenden Zeitraum gilt nach Umformung von Gleichung (1) jeweils:

$$(3) \qquad \ln(A_t / A_o) = \ln 0.95 = -k\,t = -k_{37°}\, t_{37°} = -k_{4°}\, t_{4°}$$

bzw.:

$$(4) \qquad t_{4°} / t_{37°} = k_{37°} / k_{4°}$$

Die Laufzeit bei bestimmter Temperatur ist also umgekehrt proportional zur Konstante k bei dieser Temperatur.

Für die relative Größe der Geschwindigkeitskonstanten bei verschiedenen Temperaturen hat sich die Faustregel bewährt, daß eine Erhöhung der Temperatur um 10° zu einer Verdoppelung der Geschwindigkeitskonstanten führt. Vergleichende Untersuchungen zur Stabilität von flüssigen Thromboplastinen bei verschiedenen Temperaturen liegen noch nicht vor. In einem vergleichbaren Fall (flüssiger Gerinnungsfaktors IX) wurden Beobachtungen der Degradationsgeschwindigkeitskonstanten bei verschiedenen Temperaturen von Kirkwood (Kirkwood, T.B.L.(1995) Biometrics 33, 736-742) gemacht. Er fand folgende Konstanten:

| Temperatur | k<br>$[10^{-3}$ Monat$^{-1}]$ |
|---|---|
| - 20 °C | 0.3 |
| + 4 °C | 1.0 |
| + 20 °C | 7.4 |
| + 37 °C | 30.2 |

In diesem Beispiel ist das Verhältnis der Konstanten $k_{37°} / k_4$ also ca. 30. Dies bedeutet, daß die Stabilität der Präparation bei 4°C dreißigmal größer als die Stabilität bei 37°C ist. Das heißt, daß die bei 37 °C in Tagen bestimmte Stabilität der Stabilität bei 4 °C in Monaten entspricht.

Für erfindungsgemäß hergestellte Thromboplastinzeitreagenzien liegen Belastungsdaten bei 37°C vor (siehe Beispiel 3). Bei Belastung bei 37°C ließ sich über einen Zeitraum von 30 Tagen kein Abfall der Aktivität nachweisen. Gemäß den vorangestellten Überlegungen kann daher die Stabilität der Präparation bei 4°C mit mindestens 30 Monaten angesetzt werden.

Entscheidend für ein PT-Reagenz ist aber nicht nur die Stabilität, sondern grundsätzlich auch die Sensitivität. Es ist unerwünscht, daß eine ggf. hohe Stabilität mit einem Verlust an Sensitivität erkauft werden müßte. Im Falle des erfindungsgemäßen Verfahrens konnte ein ISI-Wert von ca. 1.0, der von Experten als Ausdruck optimaler Sensitivität gesehen wird, eingestellt werden.

Die folgenden Beispiele sollen die Erfindung erläutern.

## Beispiel 1

### Herstellung eines Prothrombinzeitreagenzes

1 Teil 20 % Triton-X 100 wird mit 12 Teilen 10 % Phospholipidsuspension (Phospholipon 25 P, Nattermann, Deutschland) und 6 Teilen aufgereinigtem rekombinantem menschlichen Gewebsfaktor aus E. coli (ca. 2 mg/mL, Behring Diagnostics, Deutschland) vermischt. Nach zweistündiger Inkubation bei Raumtemperatur zur Relipidisierung wird der Ansatz mit einem 500-fachen Überschuß an Puffer verdünnt. Der Puffer besteht aus 50 mM HEPES pH 7.0, 100 mM Glyzin, 13 mM Calciumchlorid und enthält ferner folgende Stabilisatoren:

| | | |
|---|---|---|
| 2 | mM | Ascorbinsäure |
| 1 | % | Mannit |
| 1 | % | Humanserumalbumin (Behring Diagnostics, Deutschland) |

## Beispiel 2

### INR-Referenzkurve eines Prothrombinzeitreagenzes

Es wurde ein Reagenz gemäß Beispiel 1 hergestellt. Am Behring Coagulation Timer (Behring Diagnostics) wurden die Gerinnungszeiten für die AK Kalibrierplasmen (Immuno, Österreich) bestimmt. Es handelt sich um lyophilisierte Marcumarplasmen mit deklariertem INR-Wert.

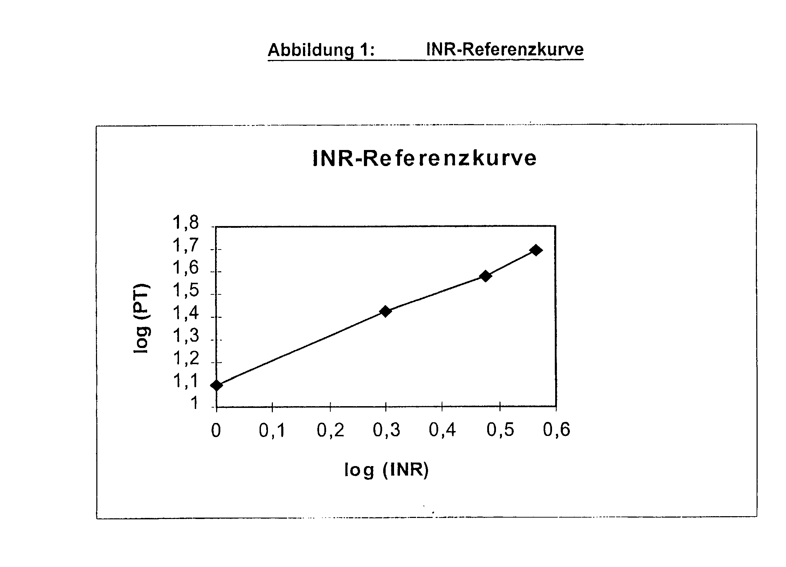

Aus den Daten können MNPT und ISI abgeschätzt werden (siehe Abb. 1)

## Beispiel 3

### Stabilität eines Prothrombinzeitreagenzes

Reagenz 1 wurde gemäß Beispiel 1 hergestellt. Reagenz 2 enthält abweichend von Beispiel 1 nur 0.1 % Humanserumalbumin. Der ISI wurde gemäß Beispiel 2 bestimmt.

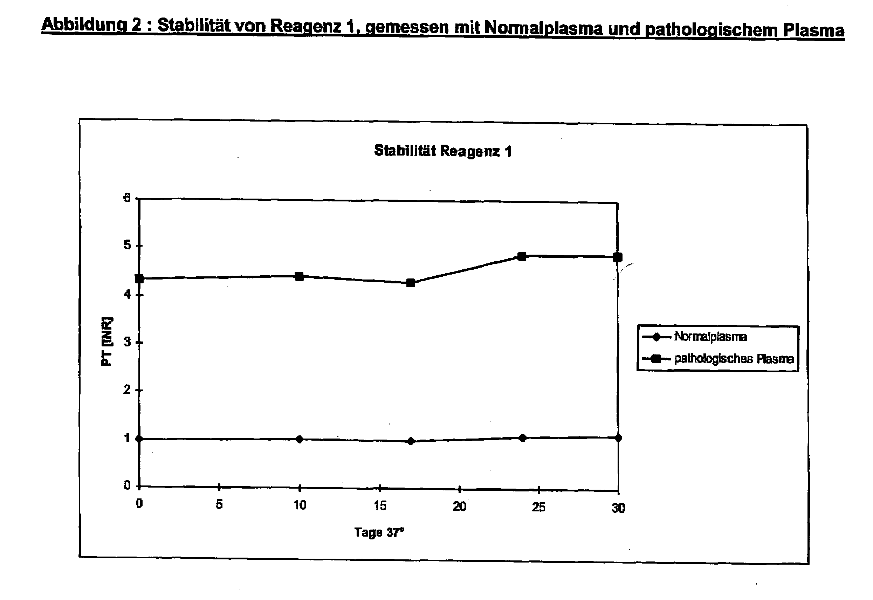

Reagenzien wurden bei 37°C belastet. Im Zeitverlauf wurden Proben genommen und die Prothrombinzeit von lyophilisiertem Normalplasma und lyophilisiertem pathologischem Plasma (Standard-Humanplasma und Pathoplasma II, Behring Diagnostics) am Behring Coagulation Timer bestimmt (siehe Abb. 2)

## Patentansprüche

1. Flüssigformulierung eines Prothrombinzeitreagenzes auf der Basis von rekombinantem Gewebsfaktor, **dadurch gekennzeichnet, daß** es mindestens ein Antioxidanz und zusätzlich ein Serumalbumin enthält.

2. Reagenz gemäß Anspruch 1, **dadurch gekennzeichnet, daß** Ascorbinsäure als Antioxidanz zugesetzt wird.

3. Reagenz gemäß Anspruch 2, **dadurch gekennzeichnet, daß** Ascorbinsäure in einer Konzentration von 1 $\mu$M bis 0.1 M verwendet wird.

4. Reagenz gemäß Anspruch 1, **dadurch gekennzeichnet, daß** das Serumalbumin humanen, tierischen oder rekombinanten Ursprungs ist.

5. Reagenz gemäß Anspruch 4, **dadurch gekennzeichnet, daß** das Serumalbumin humanen Ursprungs ist.

**6.** Reagenz gemäß Anspruch 5, **dadurch gekennzeichnet, daß** das humane Serumalbumin in einer Konzentration von 0.1 g/L bis 100 g/L zugesetzt wird.

**7.** Reagenz gemäß Anspruch 1, **dadurch gekennzeichnet, daß** der verwendete rekombinante Gewebsfaktor menschlichen oder tierischen Ursprungs ist.

**8.** Reagenz gemäß Anspruch 7, **dadurch gekennzeichnet, daß** es sich um menschlichen rekombinanten Gewebsfaktor handelt.

**9.** Reagenz gemäß Anspruch 7, **dadurch gekennzeichnet, daß** der Gewebsfaktor in Zellen menschlichen, tierischen, pflanzlichen, pilzlichen oder mikrobiellen Ursprungs kloniert und exprimiert wird.

**10.** Reagenz gemäß Anspruch 9, **dadurch gekennzeichnet, daß** die Wirtszelle Escherichia coli ist.

**Claims**

**1.** A liquid formulation of a prothrombin time reagent based on recombinant tissue factor, **characterized in that** the reagent contains at least one antioxidant and additionally a serum albumin.

**2.** The reagent as claimed in claim 1, wherein ascorbic acid is added as an antioxidant.

**3.** The reagent as claimed in claim 2, wherein ascorbic acid is used in a concentration of 1 $\mu$M to 0.1 M.

**4.** The reagent as claimed in claim 1, wherein the serum albumin is of human, animal or recombinant origin.

**5.** The reagent as claimed in claim 4, wherein the serum albumin is of human origin.

**6.** The reagent as claimed in claim 5, wherein the human serum albumin is added in a concentration of 0.1 g/l to 100 g/l.

**7.** The reagent as claimed in claim 1, wherein the recombinant tissue factor used is of human or animal origin.

**8.** The reagent as claimed in claim 7, wherein the recombinant tissue factor is of human origin.

**9.** The reagent as claimed in claim 7, wherein the tissue factor is cloned and expressed in cells of human, animal, vegetable, fungal or microbial origin.

**10.** The reagent as claimed in claim 9, wherein the host cell is Escherichia coli.

**Revendications**

**1.** Composition liquide d'un réactif pour la détermination du temps de prothrombine, à base de facteur tissulaire recombinant, **caractérisée en ce qu'**elle contient au moins un antioxydant et en outre une albumine sérique.

**2.** Réactif selon la revendication 1, **caractérisé en ce que** de l'acide ascorbique est ajouté en tant qu'antioxydant.

**3.** Réactif selon la revendication 2, **caractérisé en ce qu'**on utilise l'acide ascorbique à une concentration de 1 $\mu$M à 0,1 M.

**4.** Réactif selon la revendication 1, **caractérisé en ce que** l'albumine sérique est d'origine humaine, animale ou recombinante.

**5.** Réactif selon la revendication 4, **caractérisé en ce que** l'albumine sérique est d'origine humaine.

**6.** Réactif selon la revendication 5, **caractérisé en ce que** l'albumine sérique humaine est ajoutée à une concentration de 0,1 g/l à 100 g/l.

**7.** Réactif selon la revendication 1, **caractérisé en ce que** le facteur tissulaire recombinant qui est utilisé est d'origine humaine ou animale.

**8.** Réactif selon la revendication 7, **caractérisé en ce qu'**il s'agit d'un facteur tissulaire recombinant humain.

**9.** Réactif selon la revendication 7, **caractérisé en ce que** le facteur tissulaire est cloné et exprimé dans des cellules d'origine humaine, animale, végétale, fongique ou microbienne.

**10.** Réactif selon la revendication 9, **caractérisé en ce que** la cellule hôte est *Escherichia coli.*

**Abbildung 1:** INR-Referenzkurve

INR-Referenzkurve

# Abbildung 2 : Stabilität von Reagenz 1, gemessen mit Normalplasma und pathologischem Plasma

Stabilität Reagenz 1

EP 0 942 284 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3522148 A
- US 5385853 A
- US 5314695 A

**In der Beschreibung aufgeführte Nicht-Patentliteratur**

- **HOPPENSTAEDT, D.A. et al.** *Lab.Med.,* 1995, vol. 26 (3), 198-203
- **KIRKWOOD, T.B.L.** *Biometrics,* 1995, vol. 33, 736-742